# EUROPEAN PATENT APPLICATION

(11) **EP 1 702 617 A1**
(43) Date of publication of application: **20.09.2006**
(21) Application number: 06013196.8
(22) Date of filing: 18.10.2000
(51) Int. Cl.: A61K 31/404, A61K 31/4045, C07D 209/12, C07D 209/30, C07D 209/40, C07D 401/06

(54) **Cannabimimetic indole derivatives**

(30) Priority: 18.10.1999 US 159997 P
(62) Divisional of application: 00972266.1
(71) Applicant: The University of Connecticut, Farmington, CT 06032 (US)
(72) Inventor: Makriyannis, Alexandros, Willimantic CT 06226 (US); Deng, Hongfeng, Storrs CT 06268 (US)
(74) Representative: Beacham, Annabel Rose

(57) **Abstract**

Novel cannabimimetic indole derivatives are presented which have preferentially high affinities for one of the cannabinoid CB1 or CB2 receptor sites. The improved receptor affinity makes these analogs therapeutically useful as medications in individual and animals for treatment of pain, glaucoma, epilepsy, nausea associated with chemotherapy.

## Description

### Field of the Invention

The present invention relates generally to cannabinoid analogs and is more particularly concerned with new and improved indole cannabinoid analogs exhibiting high binding affinities for cannabinoid receptors, pharmaceutical preparations employing these analogs and methods of administering therapeutically effective amounts of the preparations to provide a physiological effect.

### Background of the Invention

Classical cannabinoids such as the marijuana derived cannabinoid △⁹-tetrahydrocannabinol, (△⁹-THC) produce their pharmacological effects via interaction with specific cannabinoid receptors in the body. So far, two cannabinoid receptors have been characterized: CB1, a central receptor found in the mammalian brain and peripheral tissues and CB2, a peripheral receptor found only in the peripheral tissues. Compounds that are agonists or antagonists for one or both of these receptors have been shown to provide a variety of pharmacological effects. See, for example, Pertwee, R.G., Pharmacology of cannabinoid CB1 and CB2 receptors, Pharmacol. Ther., (1997) 74:129 - 180 and Di Marzo, V., Melck, D., Bisogno, T., DePetrocellis, L., Endocannabinoids: endogenous cannabinoid receptor ligands with neuromodulatory action, Trends Neurosci. (1998) 21:521 - 528.

There is considerable interest in developing cannabinoid analogs possessing high affinity for one of the CB1 or CB2 receptors and/or metabolic stability. Such analogs may offer a rational therapeutic approach to a variety of disease states. One class of cannabimimetic analogs encompasses indole derivatives such as the well known aminoalkylindoles represented by WIN 55212-2 {(R)-(+)-[2,3-dihydro-5-methyl-3-[(4-morpholinyl)methyl]-pyrrolo[1,2,3-*de*]-1,4-benzoxazin-6-yl](1-naptha-lenyl)methanone}. Aminoalkylindoles of this type typically have a carbon linked alkylheterocyclic substituent at the indole-1 position, which is believed to be important for their canabimimetic activities. These known materials are not selective for preferential activation of one of the CB1 or CB2 receptors.

### Summary of the Invention

Aminoalkylindoles have been found to act as agonists for the CB1 and CB2 receptors and occasionally as antagonists for the CB1 and CB2 receptors. The invention includes compounds selective for either the CB1 or CB2 receptors. Further, some of the compounds have agonistic or antagonistic properties.

One aspect of the invention includes several novel aminoalkylindole cannabinoid analogs and physiologically acceptable salts thereof. In one embodiment of the invention, straight carbon chains were introduced to the indole-1 position. Different functional groups were also introduced to the straight carbon chains. This embodiment is shown as A.

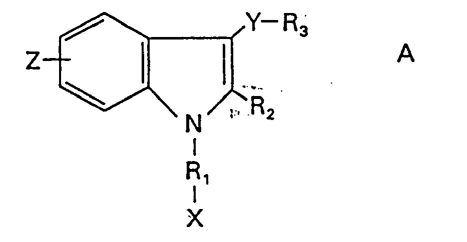

Z may be in the 4-, 5-, 6- or 7- position and is selected from the group consisting of nitro; nitroso; amino; alkylamino; dialkylamino; azido (N₃); cyano; isothiocyano and phenyl.

X is selected from the group consisting of halogen; hydrogen; hydroxy; low alkanoate; formyl; amino; cyano; isothiocyano and azido.

R₁ is selected from the group consisting of saturated or unsaturated straight carbon chains with a maximum length of seven carbon atoms; saturated or unsaturated branched carbon chains with a maximum length of seven carbon atoms; cyclic aliphatic rings interconnected to the indole-1 position with one or two carbon atoms; bicyclic aliphatic rings interconnected to the indole-1 position with one or two carbon atoms; and heterocyclic rings interconnected to the indole-1 position with one or two carbon atoms.

R₂ is selected from the group consisting of H and lower alkyl.

Y is selected from the group consisting of carbonyl and CH = CH (cis or trans).

R₃ is selected from the group consisting of phenyl; napthyl; 9-anthracenyl; phenyl with no more than two substituents selected from the group consisting of halogen, nitro, nitroso, amino, alkylamino, dialkylamino, hydroxy, methoxy, lower alkyl, azido, cyano and isothiocyano; napthyl with no more than two substituents selected from the group consisting of halogen, nitro, nitroso, amino, alkylamino, dialkylamino, hydroxy, methoxy, lower alkyl, azido, cyano and isothiocyano; and 9-anthracenyl with no more than two substituents selected from the group consisting of halogen, nitro, nitroso, amino, alkylamino, dialkylamino, hydroxy, methoxy, lower alkyl, azido, cyano and isothiocyano.

The analogs of this embodiment show high binding affinities for the CB1 and CB2 cannabinoid receptors. More importantly, some of these compounds show not only comparable cannabimimetic activity with the compound WIN 55212-2 but also a surprisingly higher selectivity for one of the CB1 or CB2 receptors. More specifically, the inventive analogs showed similar or higher receptor binding affinity than the well-known indole cannabinoid WIN 55212-2.

Another embodiment of the invention is shown as B. In this embodiment the functionalities of the novel cannabimimetic indole analogs were modified in the indole-3 and/or indole-6 positions.

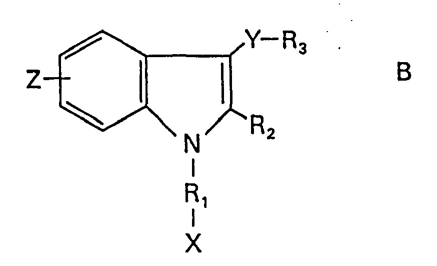

Z may be in the 4-, 5-, 6- or 7- position and is selected from the group consisting of halogen; hydroxy; methoxy and lower alkyl.

X is selected from the group consisting of hydrogen; hydroxy; lower alkanoate; formyl; amino; cyano and isothiocyano.

R₁ is selected from the group consisting of saturated or unsaturated straight carbon chains with a maximum length of seven carbon atoms; saturated or unsaturated branched carbon chains with a maximum length of seven carbon atoms; cyclic aliphatic rings interconnected to the indole-1 position with one or two carbon atoms; and bicyclic aliphatic rings interconnected to the indole-1 position with one or two carbon atoms.

R₂ is selected from the group consisting of H and lower alkyl.

Y is selected from the group consisting of carbonyl and CH = CH (cis or trans).

R₃ is selected from the group consisting of phenyl; napthyl; 9-anthracenyl; phenyl with no more than two substituents selected from the group consisting of halogen, nitro, nitroso, amino, alkylamino, dialkylamino, hydroxy, methoxy, lower alkyl, azido, cyano and isothiocyano; napthyl with no more than two substituents selected from the group consisting of halogen, nitro, nitroso, amino, alkylamino, dialkylamino, hydroxy, methoxy, lower alkyl, azido, cyano and isothiocyano, and 9-anthracenyl with no more than two substituents selected from the group consisting of halogen, nitro, nitroso, amino, alkylamino, dialkylamino, hydroxy, methoxy, lower alkyl, azido, cyano and isothiocyano.

The analogs of this embodiment are surprisingly potent cannabimimetic compounds with high CB1 and/or CB2 selectivity.

Since CB2 selective cannabinoids are able to activate the CB2 receptor and thereby modulate the immune system with little psychoactivity or other CNS effects, these analogs are possible therapeutic agents. Additionally, some of the iodide and fluoride containing analogs are potential radioactive probes for imaging *in vivo* the distribution of cannabinoid receptors. The azido modified analogs are excellent affinity probes for characterizing binding pockets of cannabinoid receptors.

The analogs disclosed herein are relatively easy to manufacture. Additionally these analogs have better physiochemical properties than naturally occurring cannabinoids. Thus, the novel cannabimimetic indole derivatives described herein, and physiologically acceptable salts thereof, represent potentially useful materials for providing a physiological effect to treat pain, peripheral pain, glaucoma, epilepsy, nausea such as associated with cancer chemotherapy, AIDS Wasting Syndrome, cancer, neurodegenerative diseases including Multiple Sclerosis, Parkinson's Disease, Huntington's Chorea and Alzheimer's Disease, mental disorders such as Schizophrenia and depression; to prevent or reduce endotoxic shock and hypotensive shock; to modulate appetite; to reduce fertility; to prevent or reduce diseases associated with motor function such as Tourette's syndrome; to prevent or reduce inflammation; to provide neuroprotection and to effect memory enhancement.

The novel cannabimimetic indole derivatives described herein also provide useful materials for testing the cannabinoid system. Thus, another aspect of the invention is the administration of a therapeutically effective amount of an inventive compound, or a physiologically acceptable salt thereof, to an individual or animal to provide a physiological effect.

### Description of Some Preferred Embodiments

As used herein, a "therapeutically effective amount" of a compound, is the quantity of a compound which, when administered to an individual or animal, results in a sufficiently high level of that compound in the individual or animal to cause a discernible increase or decrease in stimulation of cannabinoid receptors. Physiological effects that result from cannabinoid receptor stimulation include analgesia, decreased nausea resulting from chemotherapy, sedation and increased appetite. Other physiological functions include relieving intraocular pressure in glaucoma patients and suppression of the immune system. Typically, about 10 mg/day to about 1,000 mg/day is a possible "therapeutically effective amount" for the inventive compounds.

As used herein, an "individual" refers to a human. An "animal" refers to, for example, veterinary animals, such as dogs, cats, horses and the like, and farm animals, such as cows, pigs and the like.

The compound of the present invention can be administered by a variety of known methods, including orally, rectally, or by parenteral routes (e.g., intramuscular, intravenous, subcutaneous, nasal or topical). The form in which the compounds are administered will be determined by the route of administration. Such forms include, but are not limited to, capsular and tablet formulations (for oral and rectal administration), liquid formulations (for oral, intravenous, intramuscular or subcutaneous administration) and slow releasing microcarriers (for rectal, intramuscular or intravenous administration). The formulations can also contain a physiologically acceptable vehicle and optional adjuvants, flavorings, colorants and preservatives. Suitable physiologically to acceptable vehicles may include, for example, saline, sterile water, Ringer's solution, and isotonic sodium chloride solutions. The specific dosage level of active ingredient will depend upon a number of factors, including, for example, biological activity of the particular preparation, age, body weight, sex and general health of the individual being treated.

The inventive cannabinoid analogs are generally described by the structural formulas previously disclosed. The following examples are given for purposes of illustration only in order that the present invention may be more fully understood. These examples are not intended to limit in any way the practice of the invention. The prepared cannabimimetic indole derivatives can generally be described with reference to structural formulas 1 and 2 below and include physiologically acceptable salts thereof.

The inventive cannabimimetic indole derivatives of structural formula 1 include both racemics and two enantiomers.

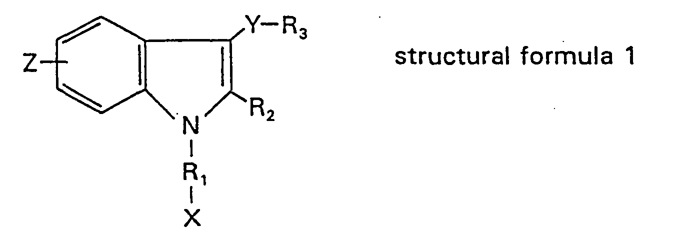

Z is in the indole-6 position and is selected from the group consisting of H; NO₂; NH₂; N₃ and NCS.

R₁ is a heterocyclic ring interconnected to the indole-1 position with one carbon atom.

X is hydrogen.

R₂ is selected from the group consisting of H and methyl.

Y is carbonyl.

R₃ is selected from the group consisting of phenyl; napthyl; adamantanyl; pyrenyl and substituted versions of any of the above.

The inventive materials of structural formula 1 are listed in TABLE 1. It should be noted that R₁ for all of the materials of TABLE 1 was 1-(N-Methyl-2-piperidinyl)methyl. All of the materials of TABLE 1 have a chiral center and the binding affinities of the materials of TABLE 1 were obtained by evaluating their racemic samples.

| TABLE 1 | | | | | |
|---|---|---|---|---|---|
| | | | | K₁ nM | |
| analog | Z | R₂ | R₃ | CB1 | CB2 |
| | | | | | |
| AM664 | NO₂ | CH₃ | 2-iodophenyl | 40 | 80.0 |
| AM665 | NH₂ | CH₃ | 2-iodophenyl | 206 | 20.3 |
| AM671 | N₃ | CH₃ | Phenyl | 155 | 59.1 |
| AM684 | NCS | CH₃ | Phenyl | 181 | 44.8 |
| AM1215 | N₃ | CH₃ | 2-iodophenyl | 40.7 | 21.9 |
| AM1216 | NCS | CH₃ | 2-iodophenyl | 210 | 25.2 |
| AM2209 | N₃ | H | 5-azido-2-iodophenyl | 48.8 | 41.8 |
| AM2223 | NCS | H | 5-isothiocyanato-2-iodophenyl | 64.8 | 29.9 |
| AM1221 | NO₃ | CH₃ | 1-naphthyl | 52.3 | 0.28 |
| AM1225 | NH₃ | CH₃ | 1-naphthyl | 439.6 | 38.5 |
| AM1231 | N₃ | CH₃ | 1-naphthyl | 31.2 | 34.2 |
| AM1218 | NO₂ | H | 1-naphthyl | 11.2 | 3.98 |
| AM1219 | NH₂ | H | 1-naphthyl | 96.6 | 31.3 |
| AM1224 | N₃ | H | 1-naphthyl | 20.2 | 0.73 |
| AM1217 | NCS | H | 1-naphthyl | 255 | 81.5 |
| AM1299 | H | H | 4-nitro-1-naphthyl | 12.4 | 13.5 |
| AM1296 | H | H | 1-naphthyl | 7.57 | 3.88 |
| AM1220 | H | H | 1-naphthyl | 3.88 | 73.4 |
| AM2212 | N₃ | H | 4-iodo-1-naphthyl | 31.0 | 2.90 |
| AM2215 | NCS | H | 4-isothiocyanato-1-naphthyl | 235 | 99.6 |
| AM1248 | H | H | adamantanyl | 100 | 332 |
| AM1253 | H | H | 2-pyrenyl | 60.3 | 126 |

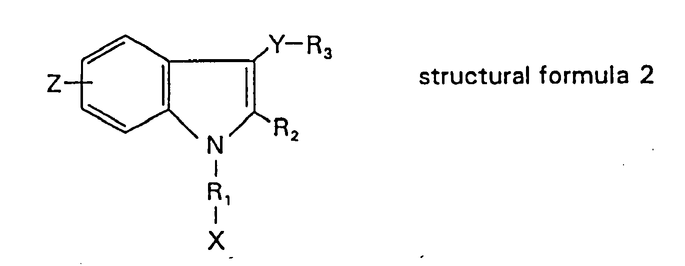

Z is in the indole-6 position and is selected from the group consisting of hydrogen; NO₂; NH₂ and halogen.

X is selected from the group consisting of halogen; H; OH; OCOCH₃; OTs; NCS; OAc and CN.

R₁ is a saturated lower alkane with a maximum length of seven carbon atoms.

R₂ is selected from the group consisting of H and methyl.

Y is carbonyl.

R₃ is selected from the group consisting of phenyl; napthyl; and substituted versions of any of the above.

The inventive materials of structural formula 1 are listed in TABLE 2. R₁ lists the number of carbon atoms in the chain at that position.

| TABLE 2 | | | | | | | |
|---|---|---|---|---|---|---|---|
| | | | | | | Kₗ nM | |
| analog | Z | R₁ | X | R₂ | R₃ | CB1 | CB2 |
| | | | | | | | |
| AM683 | H | 4 | H | CH₃ | 2-iodophenyl | 272 | 281 |
| AM669 | H | 5 | H | CH₃ | 2-iodophenyl | 47.2 | 38.6 |
| AM682 | H | 6 | H | CH₃ | 2-iodophenyl | 332 | 693 |
| AM672 | H | 7 | H | CH₃ | 2-iodophenyl | 1603 | 1511 |
| AM689 | H | 5 | OCOCH₃ | CH₃ | 2-iodophenyl | 2279 | 1019 |
| AM690 | H | 5 | OH | CH₃ | 2-iodophenyl | 4850 | 1972 |
| AM2227 | H | 5 | OTs | CH₃ | 2-iodophenyl | 1024 | 2968 |
| AM2229 | H | 5 | I | CH₃ | 2-iodophenyl | 116.5 | 46.2 |
| AM2230 | H | 5 | NCS | CH₃ | 2-iodophenyl | 195 | 29.5 |
| AM2225 | H | 5 | F | CH₃ | 2-iodophenyl | 5.97 | 3.8 |
| AM679 | H | 5 | H | H | 2-iodophenyl | 13.5 | 49.5 |
| AM692 | H | 5 | OCOCH₃ | H | 2-iodophenyl | 2656 | 1519 |
| AM693 | H | 5 | OH | H | 2-iodophenyl | 835 | 526 |
| AM697 | H | 5 | OTs | H | 2-iodophenyl | 1306 | 1116 |
| AM698 | H | 5 | I | H | 2-iodophenyl | 135.8 | 314.7 |
| AM1201 | H | 5 | NCS | H | 2-iodophenyl | 106 | 110 |
| AM694 | H | 5 | F | H | 2-iodophenyl | 0.08 | 1.44 |
| AM1202 | H | 5 | H | H | 2-iodo-5-nitrophenyl | 98.9 | 22.9 |
| AM1203 | H | 5 | H | H | 2-iodo-5-aminophenyl | 63.6 | 88.9 |
| AM1204 | H | 5 | H | H | 2-iodo-5- isothiocyanophenyl | 5659 | 3353 |
| AM1205 | H | 5 | H | H | 2-iodo-5-azidophenyl | 116.9 | 195.7 |
| AM1206 | H | 5 | H | H | 2,5-diiodophenyl | 105.1 | 150.5 |
| AM1284 | H | 3 | OCOCH₃ | H | 1-naphthyl | 126.8 | 102.8 |
| AM1289 | H | 3 | OTs | H | 1-naphthyl | 359.6 | 78.64 |
| AM1292 | H | 3 | I | H | 1-naphthyl | 3.1 | 18.1 |
| AM 1294 | H | 3 | NCS | H | 1-naphthyl | 283.3 | 237.3 |
| AM1282 | H | 4 | OCOCH₃ | H | 1-naphthyl | 133.4 | 100.8 |
| AM1283 | H | 4 | OH | H | 1-naphthyl | 117.2 | 196.5 |
| AM1286 | H | 4 | OTs | H | 1-naphthyl | 1509 | 1289 |
| AM1288 | H | 4 | I | H | 1-naphthyl | 1.3 | 10.5 |
| AM1291 | H | 4 | NCS | H | 1-naphthyl | 2958 | 1804 |
| AM1295 | H | 4 | F | H | 1-naphthyl | 2.5 | 30.7 |
| AM2232 | H | 4 | CN | H | 1-naphthyl | 0.28 | 1.48 |
| AM2231 | NO₂ | 4 | CN | H | 1-naphthyl | 4.90 | 23.9 |
| AM2202 | H | 5 | OH | H | 1-naphthyl | 33.1 | 110.6 |
| AM2203 | H | 5 | 1 | H | 1-naphthyl | 7.8 | 45.8 |
| AM2204 | H | 5 | NCS | H | 1-naphthyl | 7.5 | 24.4 |
| AM2201 | H | 5 | F | H | 1-naphthyl | 1.0 | 2.6 |
| AM1233 | NO₂ | 5 | OAc | H | 1-naphthyl | 141.7 | 153.9 |
| AM1234 | NO₂ | 5 | OH | H | 1-naphthyl | 77.6 | 196.8 |
| AM1235 | NO₂ | 5 | F | H | 1-naphthyl | 1.5 | 20.4 |
| AM1236 | NH₂ | 5 | OAc | H | 1-naphthyl | 1127 | 558.8 |
| AM1237 | NH₂ | 5 | OH | H | 1-naphthyl | 836.8 | 244.4 |
| AM1238 | I | 5 | OH | H | 1-naphthyl | 3.1 | 17.3 |
| Am1230 | I | 5 | F | H | 1-naphthyl | 1.1 | 2.4 |
| AM2210 | H | 4 | I | H | 4-nitro-1-naphthyl | 1.8 | 11.3 |
| AM2213 | H | 4 | 1 | H | 4-azido-1-naphthyl | 3.0 | 30 |
| AM2216 | H | 4 | I | H | 4-isothicocyano-1-napthyl | 42.4 | 213 |
| AM1256 | H | 5 | H | CH₃ | 4-dimethylamino-1-naphthyl | 4.74 | 18.6 |

The above materials were generally prepared as follows.

### A. General Preparation procedures for materials listed in Table 2

The materials listed in Table 2 can be prepared by methods outlined in Scheme 1.

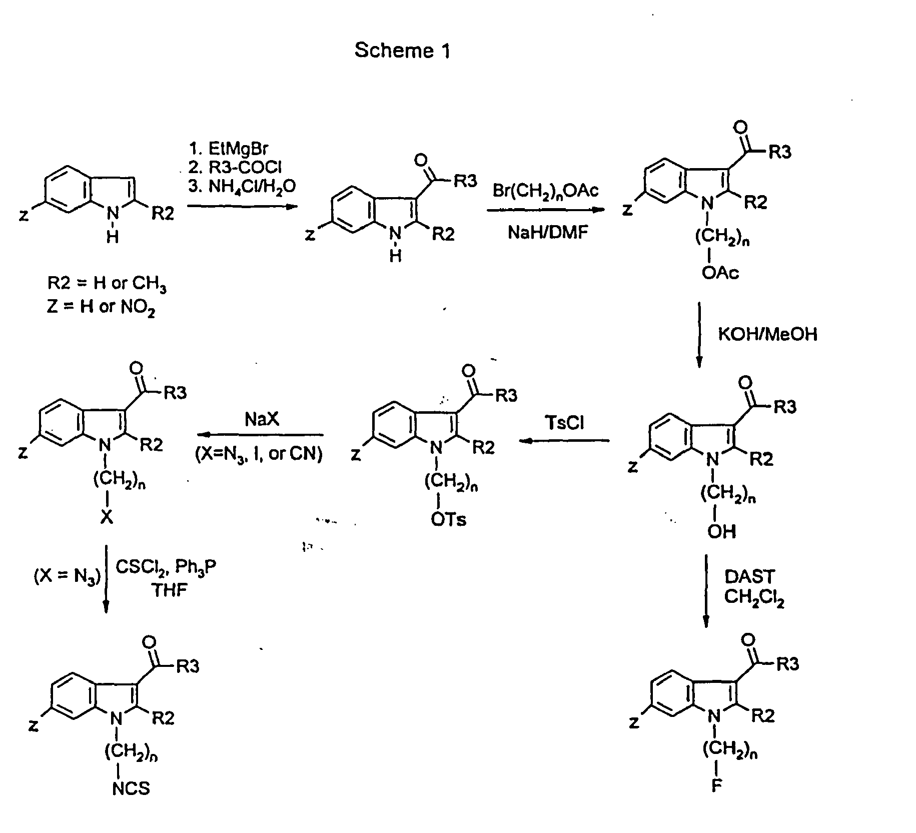

When Z = NO₂, the structures can be transformed to the different substituents as listed in Table 2 using methods outlined in Scheme 2.

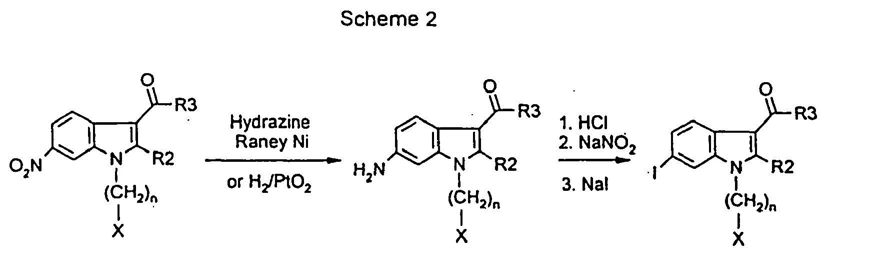

The commercially unavailable R3-COCI used in Scheme 1 can be prepared according to Scheme 3.

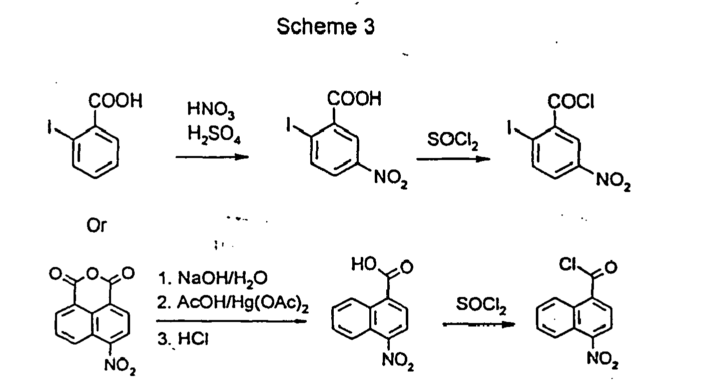

After these acid chlorides were connected to indole 3-position, the nitro group in them can be further transformed into amino, iodo, azido, and isothiocyanate groups according to the methods outlined in Scheme 4.

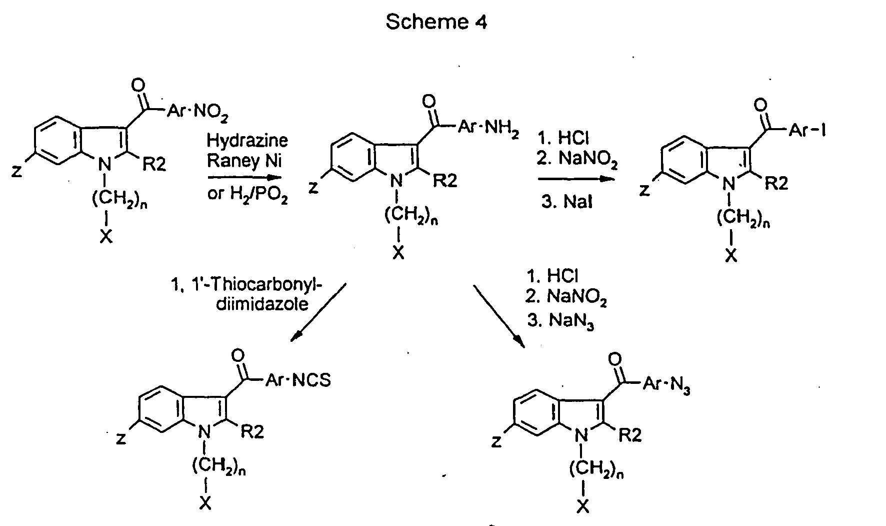

### B. General preparation procedures for materials listed in Table 1

These materials can be prepared in similarly manners as those compounds listed in Table 2 by using N-methyl-2-piperidinemethyl chloride instead of acetoxylalkylhalides for the alkylation of indole 1-position in Scheme 1.

Examples of specific analogs were prepared as follows:
3-Acyl-1*H*-indole. 17.5 ml of a 3M solution of methyl magnesium bromide in ethyl ether was added dropwise with stirring to a solution of indole (5.85 g, 50 mmol) in 50 mL of ethyl ether at 0 °C. After addition, the reaction mixture was warmed up to room temperature and stirred for 2hours (h). Then the reaction mixture was cooled down again to 0 °C and to it was added slowly with violent stirring a solution of acyl chloride (50 mmol) in 50 mL of ethyl ether. The resulting reaction mixture was warmed up to room temperature and stirred for another 1 h followed by the slow addition of 375 ml of ammonium chloride aqueous solution. After violently stirring for 30 min, a white solid was formed and filtrated. The filtrate was washed successively with ethyl ether and recrystallized from ethyl acetate:hexane to afford the product. 2-methyl-3-acyl-1*H*-indole. The foregoing procedure was repeated using 2-methyl indole in place of indole.
1-Alkyl-2-methyl-3-acyl-1*H*-indole. To a 1.2 mmol suspension of sodium hydride (48 mg, 60% in mineral oil) in 2 mL of dimethylformamide (DMF) was added 2-methyl-3-acyl-1*H*-indole (0.4 mmol). After stirring at room temperature for 30 min, alkyl bromide (0.6 mmol) was added dropwise. The resulting mixture was heated to 65 °C and stirred for 3 h followed by removal of solvent under vacuum. The residue was separated by flash column chromatography (silica gel, petroleum ether-ethyl acetate, 5:1, v/v) to afford the product.
A person of ordinary skill in the art, understanding the disclosures for the general preparation and specific preparation examples would know how to modify the disclosed procedures to achieve the above listed analogs.

The materials were tested for CB2 receptor binding affinity and for CB1 receptor affinity (to determine selectivity for the CB2 receptor). As used herein, "binding affinity" is represented by the IC₅₀ value which is the concentration of an analog required to occupy the 50% of the total number (Bmax) of the receptors. The lower the IC₅₀ value the higher the binding affinity. As used herein an analog is said to have "binding selectivity" if it has higher binding affinity for one receptor compared to the other receptor; e.g. a cannabinoid analog which has an IC₅₀ of 0.1 nM for CB1 and 10 nM for CB2, is 100 times more selective for the CB1 receptor. The binding affinities (Kᵢ) are expressed in nanomoles (nM) and are listed in TABLE 1 and TABLE 2 above.

For the CB1 receptor binding studies, membranes were prepared from rat forebrain membranes according to the procedure of P.R. Dodd et al, A Rapid Method for Preparing Synaptosomes: Comparison with Alternative Procedures, Brain Res., 107 - 118 (1981). The binding of the novel analogues to the CB1 cannabinoid receptor was assessed as described in W.A. Devane et al, Determination and Characterization of a Cannabinoid Receptor in a Rat Brain, Mol. Pharmacol., 34, 605 - 613 (1988) and A. Charalambous et al, 5'-azido Δ8 -THC: A Novel Photoaffinity Label for the Cannabinoid Receptor, J. Med. Chem., 35, 3076 - 3079 (1992) with the following changes. The above articles are incorporated by reference herein.

Membranes, previously frozen at -80°C, were thawed on ice. To the stirred suspension was added three volumes of TME (25mM Tris-HCI buffer, 5 mM MgCl₂ and 1 mM EDTA) at a pH 7.4. The suspension was incubated at 4°C for 30 min. At the end of the incubation, the membranes were pelleted and washed three times with TME.

The treated membranes were subsequently used in the binding assay described below. Approximately 30 µg of membranes were incubated in silanized 96-well microtiter plate with TME containing 0.1 % essentially fatty acid-free bovine serum albumin (BSA), 0.8 nM [³H] CP-55,940, and various concentrations of test materials at 200 °C for 1 hour. The samples were filtered using Packard Filtermate 196 and Whatman GF/C filterplates and washed with wash buffer (TME) containing 0.5% BSA. Radioactivity was detected using MicroScint 20 scintillation cocktail added directly to the dried filterplates, and the filterplates were counted using a Packard Instruments Top-Count. Nonspecific binding was assessed using 100 nM CP-55,940. Data collected from three independent experiments performed with duplicate determinations was normalized between 100% and 0% specific binding for [³H] CP-55,940, determined using buffer and 100 nM CP-55,940. The normalized data was analyzed using a 4-parameter nonlinear logistic equation to yield IC₅₀ values. Data from at least two independent experiments performed in duplicate was used to calculate IC₅₀ values which were converted to Kᵢ values using the assumptions of Cheng et al, Relationship Between the Inhibition Constant (Ki) and the concentration of Inhibitor which causes 50% Inhibition (IC50, of an Enzymatic Reaction, Biochem. Pharmacol., 22, 3099-3102, (1973), which is incorporated by reference herein.

For the CB2 receptor binding studies, membranes were prepared from frozen mouse spleen essentially according to the procedure of P.R. Dodd et al, A Rapid Method for Preparing Synaptosomes: Comparison with Alternative Procedures, Brain Res., 226, 107 - 118 (1981) which is incorporated by reference herein. Silanized centrifuge tubes were used throughout to minimize receptor loss due to adsorption. The CB2 binding assay was conducted in the same manner as for the CB1 binding assay. The binding affinities (Kᵢ) were also expressed in nanomoles (nM).

The physiological and therapeutic advantages of the inventive materials can be seen with additional reference to the following references, the disclosures of which are hereby incorporated by reference. Arnone M., Maruani J., Chaperon P, et al, Selective inhibition of sucrose and ethanol intake by SR141716, an antagonist of central cannabinoid (CB1) receptors, Psychopharmacal, (1997) 132, 104-106. Colombo G, Agabio R, Diaz G. et al: Appetite suppression and weight loss after the cannabinoid antagonist SR141716. Life Sci. (1998) 63-PL13-PL117. Simiand J, Keane M, Keane PE, Soubrie P: SR 141716, A CB1 cannabinoid receptor antagonist, selectively reduces sweet food intake in marmoset. Behav. Pharmacol (1998) 9:179-181. Brotchie JM: Adjuncts to dopamine replacement a pragmatic approach to reducing the problem of dyskinesia in Parkinson's disease. Mov. Disord. (1998) 13:871-876. Terranova J-P, Storme J-J Lafon N et al: Improvement of memory in rodents by the selective CB1 cannabinoid receptor antagonist, SR 141716. Psycho-pharmacol (1996) 126:165-172. Hampson AL Grimaldi M. Axpirod J. Wink D: Cannabidiol and (-) Δ9 tetrahydrocannabinol are neuroprotective antioxidants. Proc. Natl Acad Sci. USA (1998) 9S:8268-8273. Buckley NE, McCoy KI, Mpzey E et al Immunomodulation by cannabinoids is absent in mice deficient for the cannabinoid CB2 receptor. Eur. J Pharmacol (2000) 396:141-149. Morgan Dr: Therapeutic Uses of Cannabis. Harwood Academic Publishers, Amsterdam. (1997). Joy JE, Wagtson SJ, Benson JA: Marijuana and Medicine Assessing the Science Base. National Academy Press, Washington, DC, USA (1999). Shen M. Thayer SA: Cannabinoid receptor agonists protect cultured rat hippocampal neurons from excitotoxicity. Mol. Pharmacol (1996) 54:459-462. DePetrocellis L, Melck D, Palmisano A. et al: The endogenous cannabinoid anandamide inhibits human breaast cancer cell proliferation. Proc Natl. Acad. Sci USA (1998) 95:8375-8380. Green K. Marijuana smoking vs. cannabinoids for glaucoma therapy. Arch. Ophibalmol. (1998) feb 433-1437. Hemming M, Yellowlees PM, Effective treatment of Tourette's syndrome with marijuana. J. Psychopharmacol, (1993) 7:389-391. Muller-Vahl KB, Schneider U, Kolbe H, Emrich, HM. Treatment of Tourette's syndrome with delta-9-tetrahvdrocannabinol. Am. J. Psychiat. (1999) 156-195. Muller-Vahl KB, Kolbe H, Schneider U, Emrich, HM Cannabis in movement disorders. Porsch. Kompicmentarmed (1999) 6 (suppl. 3) 23-27. Consroe P, Musty R, Rein J, Tillery W, Pertwee R. The perceived effects of smoked cannabis on patents with multiple sclerosis, Eur. Neurol. (1997) 38-44-48. Pinnegan-Ling D, Musty R. Marinol and phantom limb pain: a case study. Proc Inv. Cannabinoid Rea. Sec. (1994):53. Brenneisen R, Pgli A, Elsohly MA, Henn V. Spiess Y: The effect of orally and rectally administered Δ9- tetrahydrocannabinol on spasticity, a pilot study with 2 patients. Int. J. Clin Pharmacol Ther. (1996) 34:446-452. Martyn CN. IIIis LS, Thom J. Nabilone in the treatment of multiple sclerosis. Lancet (1995) 345:579. Maurer M, Henn V, Dittrich A, Hofmann A. Delta-9-tetrahydrocannabinol shows antispastic and analgesic effects in a single case double-blind trial. Eur. Arch. Psychiat. Clin. Neurosci. (1990), Z40:1-4. Herzberg U, Eliav E, Bennett GJ, Kopin IJ: The analgesic effects of R(+) WIN 55,212-2 mesylate a high affinity cannabinoid agonist in a rare model of neuropathic pain. Neurosci. Letts. (1997) 221:157-160, Richardson JD, Kilo S. Hargreaves KM, Cannabinoids reduce dryperalaesia and inflammation via interaction with peripheral CB1 receptors. Pain (1998) 75:111-119. Ricardson JD, Aanonsen I, Hargreaves KM: Antihyperalgesic effects of a spinal cannabinoids. Eur. J. Pharmacol. (1998) 346:145-153. Calignano A, La Rana G. Diuffrida A, Piomelli D: Control of pain initiation by endogenous cannabinoids. Nature (1998) 394:277-291. Wagner JA, Varga K, Jarai Z, Kunos G: Mesenteric vasodilation mediated by endothelia anandamide receptors. Hypertension (1999) 33:429-434. Schuel, H., Burkman, L.J., Picone, R.P., Bo, T., Makriyannis, A., Cannabinoid receptors in human sperm. Mol. Biol. Cell., (1997) (8), 325a.

As can be seen from the results in the TABLES, some of the compounds, for example, AM1295, AM1235, AM1288 and AM694, show a high selectivity for the CB1 receptor. Other compounds, for example, AM2230, AM1289, and AM 1237, show a high selectivity for the CB2 receptor. The inventive analogs described herein, and physiologically acceptable salts thereof, have high potential when administered in therapeutically effective amounts for providing a physiological effect useful to treat pain, peripheral pain, glaucoma, epilepsy, nausea such as associated with cancer chemotherapy, AIDS Wasting Syndrome, cancer, neurodegenerative diseases including Multiple Sclerosis, Parkinson's Disease, Huntington's Chorea and Alzheimer's Disease, mental disorders such as Schizophrenia and depression; to prevent or reduce endotoxic shock and hypotensive shock; to modulate appetite; to reduce fertility; to prevent or reduce diseases associated with motor function such as Tourette's syndrome; to prevent or reduce inflammation; to provide neuroprotection and to effect memory enhancement. Thus, another aspect of the invention is the administration of a therapeutically effective amount of an inventive compound, or a physiologically acceptable salt thereof, to an individual or animal to provide a physiological effect.

In addition, some of the iodide and fluoride containing compounds, for example, AM694 and AM1230, are potential radioactive probes which would be useful for imaging *in vivo* the distribution of cannabinoid receptors. Further, azido containing compounds, for example, AM2212, AM2213 and AM1224, would be useful as affinity probes for characterizing binding pockets of cannabinoid receptors.

Those skilled in the art will recognize, or be able to ascertain with no more than routine experimentation, many equivalents to the specific embodiments of the invention disclosed herein. Such equivalents are intended to be encompassed by the scope of the invention.

## Claims

1. A compound of the formula: 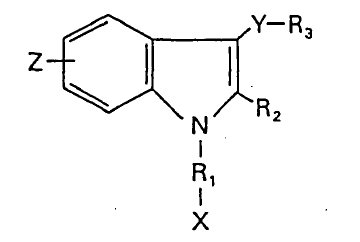 and physiologically acceptable salts thereof wherein,
Z may be in the 4-, 5-, 6- or 7- position and is selected from the group consisting of nitro, nitroso, amino, alkylamino, dialkylamino, azido, cyano, isothiocyano, and phenyl;
X is selected from the group consisting of halogen; hydrogen; hydroxy, low alkanoate, formyl, amino, cyano, isothiocyano and azido;
R₁ is selected from the group consisting of saturated or unsaturated straight carbon chains with a maximum length of seven carbon atoms, saturated or unsaturated branched carbon chains with a maximum length of seven carbon atoms, cyclic aliphatic rings interconnected to the indole-1 position with one or two carbon atoms, bicyclic aliphatic rings interconnected to the indole-1 position with one or two carbon atoms, and heterocyclic rings interconnected to the indole-1 position with one or two carbon atoms;
R₂ is selected from the group consisting of H and lower alkyl;
Y is selected from the group consisting of carbonyl and CH = CH (cis or trans); and
R₃ is selected from the group consisting of phenyl, napthyl, 9-anthracenyl, phenyl with no more than two substituents selected from the group consisting of halogen, nitro, nitroso, amino, alkylamino, dialkylamino, hydroxy, methoxy, lower alkyl, azido, cyano and isothiocyano, napthyl with no more than two substituents selected from the group consisting of halogen, nitro, nitroso, amino, alkylamino, dialkylamino, hydroxy, methoxy, lower alkyl, azido, cyano and isothiocyano and 9-anthracenyl with no more than two substituents selected from the group consisting of halogen, nitro, nitroso, amino, alkylamino, dialkylamino, hydroxy, methoxy, lower alkyl, azido, cyano and isothiocyano.

2. The compound of claim 1, wherein Z is in the indole-6 position and is selected from the group consisting of H, NO₂, NH₂ and halogen.

3. The compound of claim 1, wherein Y is C=O.

4. The compound of claim 1, wherein R1 is an alkane with a maximum length of seven carbon atoms.

5. The compound of claim 1, wherein R₂ is selected from the group consisting of H and CH₃.

6. A method of stimulating a cannabinoid receptor in an individual or animal comprising administering to the individual or animal a therapeutically effective amount of a compound having the formula: 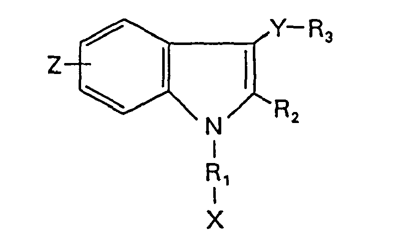 and physiologically acceptable salts thereof wherein,
Z may be in the 4-, 5-, 6- or 7- position and is selected from the group consisting of nitro, nitroso, amino, alkylamino, dialkylamino, azido, cyano, isothiocyano, and phenyl;
X is selected from the group consisting of halogen, hydrogen, hydroxy, low alkanoate, formyl, amino, cyano, isothiocyano and azido;
R₁ is selected from the group consisting of saturated or unsaturated straight carbon chains with a maximum length of seven carbon atoms; saturated or unsaturated branched carbon chains with a maximum length of seven carbon atoms; cyclic aliphatic rings interconnected to the indole-1 position with one or two carbon atoms, bicyclic aliphatic rings interconnected to the indole-1 position with one or two carbon atoms, and heterocyclic rings interconnected to the indole-1 position with one or two carbon atoms;
R₂ is selected from the group consisting of H and lower alkyl;
Y is selected from the group consisting of carbonyl and CH = CH (cis or trans); and
R₃ is selected from the group consisting of phenyl, napthyl, 9-anthracenyl, phenyl with no more than two substituents selected from the group consisting of halogen, nitro, nitroso, amino, alkylamino, dialkylamino, hydroxy, methoxy, lower alkyl, azido, cyano and isothiocyano, napthyl with no more than two substituents selected from the group consisting of halogen, nitro, nitroso, amino, alkylamino, dialkylamino, hydroxy, methoxy, lower alkyl, azido, cyano and isothiocyano and 9-anthracenyl with no more than two substituents selected from the group consisting of halogen, nitro, nitroso, amino, alkylamino, dialkylamino, hydroxy, methoxy, lower alkyl, azido, cyano and isothiocyano.

7. A compound of the formula: 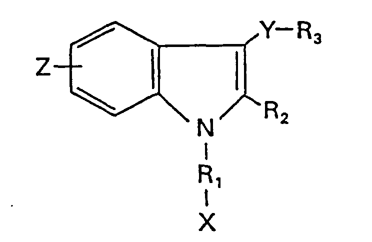 and physiologically acceptable salts thereof wherein,
Z may be in the 4-, 5-, 6- or 7- position and is selected from the group consisting of halogen, hydroxy, methoxy, and lower alkyl;
X is selected from the group consisting of halogen, hydrogen, hydroxy, lower alkanoate, formyl, cyano, and isothiocyano;
R₁ is selected from the group consisting of saturated or unsaturated straight carbon chains with a maximum length of seven carbon atoms, saturated or unsaturated branched carbon chains with a maximum length of seven carbon atoms, cyclic aliphatic rings interconnected to the indole-1 position with one or two carbon atoms and bicyclic aliphatic rings interconnected to the indole-1 position with one or two carbon atoms;
R₂ is selected from the group consisting of H and lower alkyl;
Y is selected from the group consisting of carbonyl and CH=CH (cis or trans); and
R₃ is selected from the group consisting of phenyl, napthyl, 9-anthracenyl, phenyl with no more than two substituents selected from the group consisting of halogen, nitro, nitroso, amino, alkylamino, dialkylamino, hydroxy, methoxy, lower alkyl, azido, cyano and isothiocyano, napthyl with no more than two substituents selected from the group consisting of halogen, nitro, nitroso, amino, alkylamino, dialkylamino, hydroxy, methoxy, lower alkyl, azido, cyano and isothiocyano, and 9-anthracenyl with no more than two substituents selected from the group consisting of hatogen, nitro, nitroso, amino, alkylamino, dialkylamino, hydroxy, methoxy, lower alkyl, azido, cyano and isothiocyano.

8. The compound of claim 7, wherein Z is in the indole-6 position and is selected from the group consisting of H, NO₂, NH₂, N₃ and NCS.

9. The compound of claim 7, wherein R₁ is CH₂ and X is a heterocyclic structure.

10. The compound of claim 7, wherein Y is C=O.

11. The compound of claim 7, wherein R₂ is selected from the group consisting of H and CH₃.

12. A method of stimulating a cannabinoid receptor in an individual or animal comprising administering to the individual or animal a therapeutically effective amount of a compound having the formula: 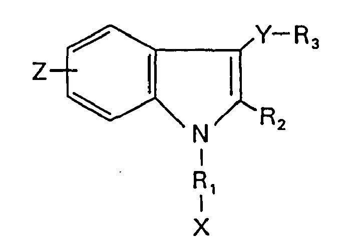 and physiologically acceptable salts thereof wherein,
Z may be in the 4-, 5-, 6- or 7- position and is selected from the group consisting of halogen, hydroxy, methoxy, and lower alkyl;
X is selected from the group consisting of halogen, hydrogen, hydroxy, lower alkanoate, formyl, cyano, and isothiocyano;
R₁ is selected from the group consisting of saturated or unsaturated straight carbon chains with a maximum length of seven carbon atoms, saturated or unsaturated branched carbon chains with a maximum length of seven carbon atoms, cyclic aliphatic rings interconnected to the indole-1 position with one or two carbon atoms and bicyclic aliphatic rings interconnected to the indole-1 position with one or two carbon atoms;
R₂ is selected from the group consisting of H and lower alkyl;
Y is selected from the group consisting of carbonyl and CH = CH (cis or trans); and
R₃ is selected from the group consisting of phenyl, napthyl, 9-anthracenyl, phenyl with no more than two substituents selected from the group consisting of halogen, nitro, nitroso, amino, alkylamino, dialkylamino, hydroxy, methoxy, lower alkyl, azido, cyano and isothiocyano, napthyl with no more than two substituents selected from the group consisting of halogen, nitro, nitroso, amino, alkylamino, dialkylamino, hydroxy, methoxy, lower alkyl, azido, cyano and isothiocyano, and 9-anthracenyl with no more than two substituents selected from the group consisting of halogen, nitro, nitroso, amino, alkylamino, dialkylamino, hydroxy, methoxy, lower alkyl, azido, cyano and isothiocyano.

13. A pharmaceutical preparation containing a therapeutically effective amount of a compound having the formula: 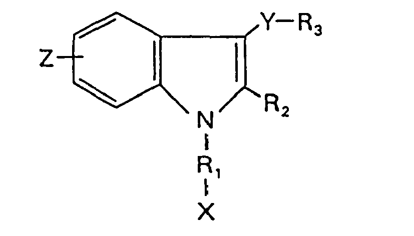 and physiologically acceptable salts thereof wherein,
Z may be in the 4-, 5-, 6- or 7- position and is selected from the group consisting of nitro, nitroso, amino, alkylamino, dialkylamino, azido, cyano, isothiocyano, and phenyl;
X is selected from the group consisting of halogen; hydrogen; hydroxy, low alkanoate, formyl, amino, cyano, isothiocyano and azido;
R₁ is selected from the group consisting of saturated or unsaturated straight carbon chains with a maximum length of seven carbon atoms, saturated or unsaturated branched carbon chains with a maximum length of seven carbon atoms, cyclic aliphatic rings interconnected to the indole-1 position with one or two carbon atoms, bicyclic aliphatic rings interconnected to the indole-1 position with one or two carbon atoms, and heterocyclic rings interconnected to the indole-1 position with one or two carbon atoms;
R₂ is selected from the group consisting of H and lower alkyl;
Y is selected from the group consisting of carbonyl and CH = CH (cis or trans); and
R₃ is selected from the group consisting of phenyl, napthyl, 9-anthracenyl, phenyl with no more than two substituents selected from the group consisting of halogen, nitro, nitroso, amino, alkylamino, dialkylamino, hydroxy, methoxy, lower alkyl, azido, cyano and isothiocyano, napthyl with no more than two substituents selected from the group consisting of halogen, nitro, nitroso, amino, alkylamino, dialkylamino, hydroxy, methoxy, lower alkyl, azido, cyano and isothiocyano and 9-anthracenyl with no more than two substituents selected from the group consisting of halogen, nitro, nitroso, amino, alkylamino, dialkylamino, hydroxy, methoxy, lower alkyl, azido, cyano and isothiocyano.

14. The pharmaceutical preparation of claim 13 wherein,
Z may be in the 4-, 5-, 6- or 7- position and is selected from the group consisting of halogen, hydroxy, methoxy, and lower alkyl;
X is selected from the group consisting of halogen, hydrogen, hydroxy, lower alkanoate, formyl, cyano, and isothiocyano;
R₁ is selected from the group consisting of saturated or unsaturated straight carbon chains with a maximum length of seven carbon atoms, saturated or unsaturated branched carbon chains with a maximum length of seven carbon atoms, cyclic aliphatic rings interconnected to the indole-1 position with one or two carbon atoms and bicyclic aliphatic rings interconnected to the indole-1 position with one or two carbon atoms;
R₂ is selected from the group consisting of H and lower alkyl;
Y is selected from the group consisting of carbonyl and CH = CH (cis or trans); and
R₃ is selected from the group consisting of phenyl, napthyl, 9-anthracenyl, phenyl with no more than two substituents selected from the group consisting of halogen, nitro, nitroso, amino, alkylamino, dialkylamino, hydroxy, methoxy, lower alkyl, azido, cyano and isothiocyano, napthyl with no more than two substituents selected from the group consisting of halogen, nitro, nitroso, amino, alkylamino, dialkylamino, hydroxy, methoxy, lower alkyl, azido, cyano and isothiocyano, and 9-anthracenyl with no more than two substituents selected from the group consisting of halogen, nitro, nitroso, amino, alkylamino, dialkylamino, hydroxy, methoxy, lower alkyl, azido, cyano and isothiocyano.
